# EUROPEAN PATENT APPLICATION

(11) **EP 2 193 794 A1**
(43) Date of publication of application: **09.06.2010**
(21) Application number: 09015073.1
(22) Date of filing: 04.12.2009
(51) Int. Cl.: A61K 31/496, A61K 45/06, A61P 25/16

(54) **Use of eltoprazine for the treatment of L-Dopa-induced dyskinesia**

(30) Priority: 05.12.2008 US 201027 P; 06.02.2009 EP 09001689; 30.06.2009 US 269858 P
(71) Applicant: Merz Pharma GmbH & Co. KGaA, 60318 Frankfurt am Main (DE)
(72) Inventor: Valastro, Barbara, Dr., 60325 Frankfurt am Main (DE); Dekundy, Andrzej, Dr., 61137 Schöneck (DE); Tennigkeit, Frank, Dr., 55218 Ingelheim (DE); Russ, Hermann, Prof.Dr.Dr., 60594 Frankfurt am Main (DE); Franke, Lutz, Dr., 61118 Bad Vilbel (DE)
(74) Representative: Ricker, Mathias

(57) **Abstract**

The present invention relates to the treatment of an individual afflicted with L-DOPA-induced dyskinesia, which condition may arise as a consequence of long-term treatment with L-DOPA therapy in Parkinson's patients, the instant treatment comprising administering to the individual an effective amount of eltoprazine or a pharmaceutically acceptable salt thereof.

## Description

### FIELD OF THE INVENTION

The present invention relates to the efficient treatment of an individual afflicted with L-DOPA-induced dyskinesia, which condition typically arises as a consequence of long-term treatment with L-DOPA therapy in Parkinson patients, the instant treatment comprising administering to the individual an effective amount of eltoprazine or a pharmaceutically acceptable salt thereof.

### BACKGROUND OF THE INVENTION

This invention relates to an innovative method of treating patients afflicted with L-DOPA-induced dyskinesia, which condition may arise as a consequence of long-term treatment with L-DOPA therapy in Parkinson patients.

Parkinsonian symptoms are characterized by slowness of movement (bradykinesia/akinesia), rigidity and/or tremor. These symptoms may have an idiopathic, toxic, traumatic or genetic origin (e.g., in Parkinson's disease (PD)) or may also occur as a consequence of treatment, e.g., with dopamine receptor antagonists in schizophrenia (Parkinson syndrome).

Currently, the principal symptomatic treatment for PD is based upon administration of dopamine-replacement therapy (e.g., levodopa (L-DOPA)) and/or therapy with dopamine receptor agonists (e.g., apomorphine). Long-term treatment with either therapy may lead to the development of dyskinesia, which is the most important motor complication that may arise in Parkinson patients. Although patients treated with dopamine receptor agonists are less prone to develop severe dyskinesia, other non-motor side effects such as somnolence, constipation, dizziness, nausea and hallucination have been reported to be increased with this treatment option. Concerning L-DOPA, long-term use of this treatment option leads to a reduction of its anti-parkinsonian efficacy and to the development of L-DOPA-induced dyskinesia (LID).

LID is characterised by a mixture of choreiform, dystonic or ballistic/myoclonic movements that are observed after L-DOPA administration. It is reported that about 30% of the Parkinson patients will experience dyskinesia after 4-6 years of treatment with L-DOPA while close to 90% will suffer from this complication after 9 years. Although the cause of dyskinesia remains unknown, the main risk factor for the development of LID is young age at PD onset, the disease severity and duration as well as a high initial dose of L-DOPA treatment. Ultimately, this complication severely impairs the quality of life and well-being of the patient and therefore limits the use of this drug as most important therapeutic agent.

According to the literature, LID may be related to the pulsatile and intermittent nature of L-DOPA therapy. Upon systemic administration of L-DOPA to Parkinson patients, dopamine is formed, stored and released by the remaining dopaminergic terminals as well as by other cellular components present in the striatum such as serotoninergic neurons. Since the striatal serotonin system remains relatively spared in most PD patients, it is believed to play an important role in determining the efficacy of L-DOPA therapy.

Many attempts have been made to develop serotonergic agents for the prevention or treatment of LID but these attempts have had limited success in patients. Based on the clinical results obtained with different 5-HT1 a agonists such as buspirone, sarizotan and tandospirone, it becomes evident that the 5-HT1 a component is not sufficient to treat the development of LID in Parkinson's patients in a way that makes a meaningful difference for patients. According to a recently published study, it is now suggested that the combination of a 5-HT1 a agonist with a 5-HT1 b agonist could prevent the dysregulated release of dopamine by serotonin neurons and therefore be beneficial for the treatment of LID (Carta M, Carlsson T, Kirik D and Björklund A. Dopamine released from 5-HT terminals is the cause of L-DOPA-induced dyskinesia in Parkinsonian rats, Brain 2007 130: 1819-1833). Although this combination has shown some positive results in the rat model of dyskinesia, the administration of Ru-24969, a substance acting through the joint stimulation of the 5-HT1a and 5-HT1 b receptors, was associated with worsening of motor symptoms and/or development of side effects in monkey studies (Iravani MM, Jackson MJ, Kuoppamäki M, Smith LA, Jenner P. 3,4-methylenedioxymethamphetamine (ecstasy) inhibits dyskinesia expression and normalizes motor activity in 1-methyl-4-phenyl-1,2,3,6-tetrahydropyridine-treated primates, J Neurosci. 2003 23:9107-9115). These observations have prohibited its further development for the treatment of Parkinson patients with LID.

The present invention relates to the use of eltoprazine and its salts, solvates and conjugates, which we have determined possesses a unique receptor profile. Consequently, the present invention relates to the use of eltoprazine for the treatment of Parkinson patients with motor complications. Besides the strong antidyskinetic efficacy, eltoprazine also provides surplus benefits by improving co-morbidities typically seen in PD patients, which could lead to a dramatic improvement of the quality-of-life of Parkinson patients.

### SUMMARY OF THE INVENTION

The present invention relates to a method of treating or preventing L-DOPA-induced dyskinesia in a subject in need thereof, comprising administering an effective amount of eltoprazine or a pharmaceutically acceptable salt thereof.

A further aspect of the invention relates to such a method comprising administering an effective amount of eltoprazine hydrochloride.

A further aspect of the invention relates to such a method wherein eltoprazine is administered in a range from about 5 mg to about 75 mg/day.

A further aspect of the invention relates to such a method wherein eltoprazine is administered in a range from about 5 mg to about 50 mg/day.

A further aspect of the invention relates to such a method wherein eltoprazine is administered in a range from about 5 mg to about 60 mg/day.

A further aspect of the invention relates to such a method wherein eltoprazine is administered in a range from about 5 mg to about 40 mg/day.

A further aspect of the invention relates to such a method wherein eltoprazine is administered in a range from about 5 mg to about 20 mg/day.

A further aspect of the invention relates to such a method wherein eltoprazine is administered at about 20 mg/day.

A further aspect of the invention relates to such a method wherein eltoprazine is administered at about 40 mg/day.

A further aspect of the invention relates to such a method wherein eltoprazine is administered at about 60 mg/day.

A further aspect of the invention relates to such a method wherein eltoprazine is administered between about 10 and 15 mg/day.

A further aspect of the invention relates to such a method wherein eltoprazine or a pharmaceutically acceptable salt thereof is administered once a day, twice a day (b.i.d.), or three times a day.

A further aspect of the invention relates to such a method wherein eltoprazine is administered in an oral formulation.

A further aspect of the invention relates to such a method wherein the subject suffers from a movement disorder.

A further aspect of the invention relates to such a method wherein the movement disorder is parkinsonism.

A further aspect of the invention relates to such a method wherein the parkinsonism is idiopathic (PD).

A further aspect of the invention relates to such a method wherein the subject is undergoing long-term treatment with L-DOPA therapy indicated for the treatment of PD.

A further aspect of the invention relates to such a method wherein the subject is undergoing treatment with dopamine receptor agonists.

A further aspect of the invention relates to a composition comprising eltoprazine or a pharmaceutically acceptable salt thereof (e.g., eltoprazine hydrochloride) for the treatment or prevention of L-DOPA-induced dyskinesia.

A further aspect of the invention relates to the use of eltoprazine or a pharmaceutically acceptable salt thereof (e.g., eltoprazine hydrochloride) for the manufacture of a medicament for the treatment or prevention of L-DOPA-induced dyskinesia.

A further aspect of the invention relates to the above-defined composition or use wherein eltoprazine or a pharmaceutically acceptable salt thereof (e.g., eltoprazine hydrochloride) is for administration in a range from about 5 mg to about 150 mg/day, or in a range from about 5 mg to about 100 mg/day, or in a range from about 5 mg to about 75 mg/day, in a range from about 5 mg to about 75 mg/day, or in a range from about 5 mg to about 60 mg/day, or in a range from about 5 mg to about 50 mg/day, or in a range from about 5 mg to about 40 mg/day, or in a range from about 5 mg to about 20 mg/day, or in a range from about 5 mg to about 15 mg/day, or wherein eltoprazine is for administration at about 10 mg/day, wherein eltoprazine is for administration at about 15 mg/day, is for administration at about 20 mg/day, or eltoprazine is for administration at about 40 mg/day, or eltoprazine is for administration at about 60 mg/day, or eltoprazine is for administration at about 80 mg/day.

A further aspect of the invention relates to the above-defined composition or use wherein eltoprazine or a pharmaceutically acceptable salt thereof (e.g., eltoprazine hydrochloride) is for administration once a day, twice a day (b.i.d.), or three times a day.

A further aspect of the invention relates to the above-defined composition or use wherein eltoprazine or a pharmaceutically acceptable salt thereof (e.g., eltoprazine hydrochloride) is for administration in an oral formulation.

A further aspect of the invention relates to the above-defined composition or use wherein the subject to be treated suffers from a movement disorder.

A further aspect of the invention relates to the above-defined composition or use wherein the movement disorder is parkinsonism.

A further aspect of the invention relates to the above-defined composition or use wherein the parkinsonism is idiopathic (PD).

A further aspect of the invention relates to the above-defined composition or use wherein the subject is undergoing long-term treatment with L-DOPA therapy indicated for the treatment of PD.

A further aspect of the invention relates to the above-defined composition or use wherein the subject is undergoing treatment with dopamine receptor agonists.

A further aspect of the invention relates to a method comprising administering a therapeutically effective amount of eltoprazine or a pharmaceutically acceptable salt thereof in combination with at least one additional pharmaceutical agent which has been shown to be effective for the treatment of PD.

### BRIEF DESCRIPTION OF THE DRAWINGS

Figure 1 shows the potency of eltoprazine on different serotonin receptors at relevant plasma concentration (< 0.5µM).

Figure 2 shows the effect of eltoprazine on AIM scores in unilaterally 6-OHDA-lesioned rats.

Figure 3 shows the effect of eltoprazine on turning behavior induced by high dose of L-DOPA in unilaterally 6-OHDA-lesioned rats.

### DETAILED DESCRIPTION OF THE INVENTION

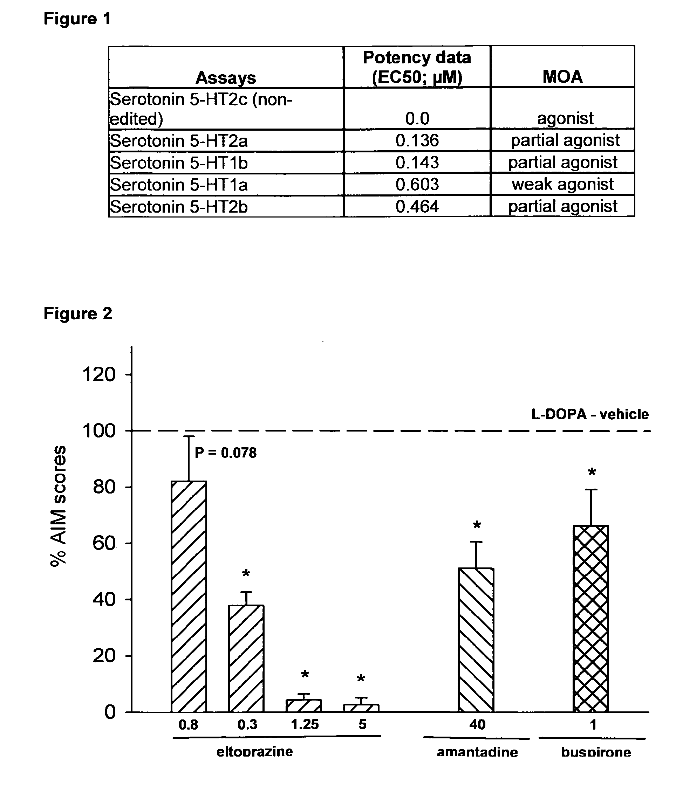

The peculiarity of this invention compared to former treatment approaches for L-DOPA-induced dyskinesia is the so far unknown therapeutic efficiency of eltoprazine, which is based on a unique receptor profile with several components potentiating each other in an unexpected way. Although the pharmacological action of eltoprazine was mainly attributed to its agonistic effect on the 5-HT1 a and 5-HT1 b receptors (see Schipper J, Tulp MTM, Sijbesma H. Neurochemical profile of eltoprazine. Drug metabolism and Drug interactions 1990 8:85-114), we have observed that eltoprazine acts as an agonist at the 5-HT2c receptor in human recombinant cell lines, in addition to its partial agonistic action on 5-HT2a and 5-HT1 b receptors as well as its weak agonistic action on the 5-HT1 a receptor (Figure 1).

In Parkinson patients with LID, 5-HT2c receptor binding was found to be increased in the output structures of the basal ganglia, which led to the speculation that a reduction in 5-HT2c transmission could contribute to the development of LID (Fox SH, Brotchie, JM. 5-HT2c receptor binding is increased in the substantia nigra pars reticulate in Parkinson's disease, Mov Disord. 2000 15:1064-1069). Since eltoprazine acts as a 5-HT2c and partial 5-HT2a agonist, we believe that the simultaneous activation of these postsynaptic receptors on the output structures and the stimulation of the presynaptic 5-HT1 a and 5-HT1 b receptors located in the striatum would prevent 1) the inhibitory drive on the thalamus seen in LID as well as 2) the dysregulated release of dopamine produced by the conversion of L-DOPA in intact serotonergic neurons. In addition, the use of a 5-HT2c and 5-HT2a receptor agonist could compensate for the reduction of physiological serotonin release that may be caused by the activation of the presynaptic 5-HT1 a and 5-HT1 b receptors by eltoprazine. Based on this hypothesis, we believe that the stimulation of this unique and novel target combination contributes to the overall beneficial effect of eltoprazine in LID (Figure 2).

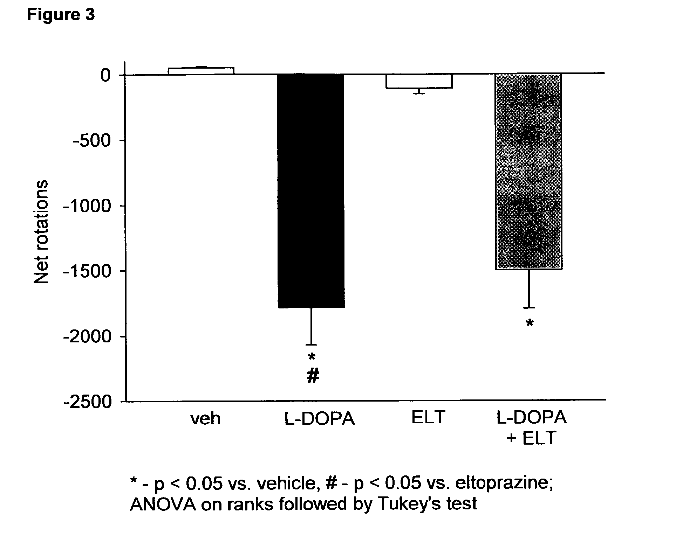

The present invention relates to the use of eltoprazine and its salts, solvates and conjugates, which possesses a unique receptor profile targeting the 5-HT2a, 5-HT2c, 5-HT1 a and 5-HT1 b receptors at relevant plasma concentration. Furthermore, eltoprazine is devoid of significant affinity for the dopamine D2 receptor and does not interfere with the anti-parkinsonian effect of L-DOPA (Figure 3), which constitutes another advantage for LID. Although the activation of individual serotonergic receptors has been investigated for the treatment of LID, the concomitant and synergistic action of a single molecule on the 5-HT2c, 5-HT2a, 5-HT1 b and 5-HT1 a receptors, in addition to a lack of significant dopaminergic binding, produces a so far unknown strong antidyskinetic effect without adverse events such as worsening of the underlying Parkinson's disease. In theory, the unique efficacy could be achieved also through a cocktail of drugs mimicking these composite effects, however, such a cocktail of several molecules cannot successfully be developed in a clinical setting obeying regulatory requirements.

In addition to this strong antidyskinetic effect, eltoprazine
- improves the primary symptoms of PD (bradykinesia, OFF time etc.),
- improves depression and anxiety, which are one of the most challenging co-morbidities in Parkinson's disease
- reduces pain, which is an extremely inconvenient consequence of dystonic dyskinesia as well as of muscular rigidity
- improves "on / off phenomenon"

Some of these effects have been demonstrated in relevant animal models.

Thus, in a particular embodiment the present invention relates to a method for administering an effective amount of eltoprazine or a pharmaceutically acceptable salt thereof for improving the primary symptoms of PD, such as bradykinesia an/or OFF time, in a subject having Parkinson's disease, particularly to a subject suffering from L-DOPA induced dyskinesia, and to compositions comprising eltoprazine or a pharmaceutically acceptable salt thereof for use in such methods.

Furthermore, in another particular embodiment the present invention relates to a method for administering an effective amount of eltoprazine or a pharmaceutically acceptable salt thereof for improving depression and/or anxiety in a subject having Parkinson's disease, particularly to a subject suffering from L-DOPA induced dyskinesia, and to compositions comprising eltoprazine or a pharmaceutically acceptable salt thereof for use in such methods.

Compounds with 5-HT1a agonistic activity such as Tandospirone citrate, Gepirone hydrochloride or Ipsapirone hydrochloride are already approved or are currently tested for use in the treatment of depression and/or anxiety. Additionally, compounds having a 5-HT2c agonistic activity, such as Org 37684, Ro 60-0175, YM348, WAY-163909, or mCPP, have shown positive effects in rodent models of depression. Thus, eltoprazine with its unique receptor profile should be ideally suited for addressing depression in PD patients by (i) preventing and/or treating LID events that may contribute to the development and progression of depression, (ii) directly acting as agonist on both 5-HT1 a and 5-HT2c receptors implicated in depression, and (iii) acting as agonist on 5-HT1 b receptor implicated in the reduction of impulsivity and improvement of the reward mechanism (van den Bergh et al., Pharmacol Biochem Behav. 85 (2006) 736-43. Epub 2007 Jan 17).

Furthermore, in another particular embodiment the present invention relates to a method for administering an effective amount of eltoprazine or a pharmaceutically acceptable salt thereof for improving pain in a subject having Parkinson's disease, particularly to a subject suffering from pain as a consequence of dystonic dyskinesia as well as of muscular rigidity, and to compositions comprising eltoprazine or a pharmaceutically acceptable salt thereof for use in such methods.

As used herein, the term "subject" encompasses mammals including animals and humans.

The term eltoprazine is known in the art and may also be known as DU-28853 and 1-(2,3-dihydro-1,4-benzodioxin-5-yl)piperazine. As used herein, eltoprazine refers to the substance, as well as its pharmaceutically acceptable salts.

The term "agonist" refers to a substance that binds to a receptor and mimics the cellular effect of the native or endogenous ligand for the same receptor. The term agonist includes the class of agents called full agonists, which bind and display full efficacy at the receptor, and partial agonists, which have only partial efficacy at the receptor. Partial agonists may also be seen as competitive antagonists, competing away the endogenous ligand when it is in excess or give a sub maximal response when inadequate amount of endogenous ligand is present. The term "activation" refers to the state of a receptor when an agonist is bound to it.

The term "dyskinesia" refers to involuntary movements similar to a tic or chorea, which interferes with the performance of voluntary movements. The term dyskinesia may be used in relation to Parkinson's disease and/or other extrapyramidal disorders. In the case of Parkinson's disease, dyskinesia may occur as a complication of dopaminomimetic therapy with L-DOPA (LID), or, in rare cases, after treatment with dopamine receptor agonists such as apomorphine. In LID, dyskinesia most commonly occurs in two forms: peak dose, also known as improvement-dyskinesia-improvement (IDI), which most commonly occur at the time of peak L-DOPA plasma concentrations, and diphasic dyskinesia also known as dyskinesia-improvement-dyskinesia (DID), which occur when the L-DOPA concentration rises or falls.

The term "levodopa" or "L-DOPA" refers to a therapeutic strategy use to treat PD. L-DOPA is defined as a dopamine precursor because L-DOPA needs to be decarboxylated to dopamine to be effective. On the other hand, the term "dopamine receptor agonist" refers to dopamine D1, D2 or D3 receptor agonists, which bind directly to their respective receptors to induce an effect.

The term "treat" is used herein to mean to relieve or alleviate at least one symptom of a disease in a subject. Within the meaning of the present invention, the term "treat" also denotes to arrest, delay the onset (*i.e*., the period prior to clinical manifestation of a disease) and/or reduce the risk of developing or worsening a disease.

The term "therapeutically effective" applied to dose or amount refers to that quantity of a compound or pharmaceutical composition sufficient to result in a desired activity upon administration to a mammal in need thereof.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans.

The term "salt" is defined as a chemical containing different charged components. The term salt also includes hydrates and solvates. Contemplated in the instant description are pharmaceutically acceptable salts, which salts may include, but are not limited to, acid addition salts, such as those made with hydrochloric, sulphuric, nitric, phosphoric, acetic, maleic, fumaric, tartaric, citric, benzoic, methane sulphonic, naphthalene sulphonic, p-toluene sulphonic acid. All of these salts (or other similar salts) may be prepared by conventional means. The nature of the salt is not critical, provided that it is non-toxic and does not substantially interfere with the desired pharmacological activity.

Eltoprazine may be used according to the invention in the form of any of pharmaceutically acceptable salts, solvates and conjugates. Any references to eltoprazine in this description should be understood as also referring to such salts, solvates and conjugates.

The term "carrier" applied to pharmaceutical compositions of the invention refers to a diluent, excipient, or vehicle with which an active compound (e.g., eltoprazine) is administered. Such pharmaceutical carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

The term "about" or "approximately" usually means within 20%, alternatively within 10%, including within 5% of a given value or range. Alternatively, especially in biological systems, the term "about" means within about a log (*i.e.,* an order of magnitude), including within a factor of two of a given value.

In conjunction with the methods of the present invention, also provided are pharmaceutical compositions comprising a therapeutically effective amount of eltoprazine. The compositions of the invention may further comprise a carrier or excipient (all pharmaceutically acceptable). The compositions may be formulated e.g. for once-a-day administration, twice-a-day administration, or three times a day administration.

The active ingredient (e.g., eltoprazine) or the composition of the present invention may be used for the treatment of at least one of the mentioned disorders, wherein the treatment is adapted to or appropriately prepared for a specific administration as disclosed herein (e.g., to once-a-day, twice-a-day, or three times a day administration). For this purpose the package leaflet and/or the patient information contains corresponding information.

In one embodiment, a therapeutically effective amount of eltoprazine is administered before the administration of a therapeutically effective amount of L-DOPA, for example at least about 10, 15, 20, 25, 30, 35, or 40 minutes before the administration of a therapeutically effective amount of L-DOPA. In particular embodiments, a therapeutically effective amount of eltoprazine is administered between about 10 and 25 minutes before the administration of a therapeutically effective amount of L-DOPA. This administration scheme should allow synchronizing the peak plasma level of L-DOPA with eltoprazine plasma levels, due to the faster uptake of L-DOPA in comparison to eltoprazine. In doing so, the brain-activity of both substances should occur during the same time period.

In another embodiment, a therapeutically effective amount of eltoprazine is administered twice per day. In particular, eltoprazine is administered once in the morning, particularly once prior to the first L-DOPA administration, and once in the middle of the day, particularly at about lunchtime, wherein the lunchtime treatment is between about 6 and 10, particularly between about 7 and 9 hours before the patient wishes to go to bed, or, alternatively, prior to the mid-point of daily L-DOPA dosages (e.g. prior to the third L-DOPA dosage in a five-time-daily L-DOPA dosage regimen, or between the third and fourth L-DOPA dosages in a six-time-daily L-DOPA dosage regimen). Such an administration should allow for the eltoprazine plasma level to ebb away during evening and at nighttime, reducing the risk of an impaired REM-(*rapid eye movement*)-activity.

In certain such embodiments, the treatment is the treatment of diphasic dyskinesia (DID).

In another embodiment, the first dosage of eltoprazine consist of about 55 to 65% of the total daily dosage amount, and the second dose of eltoprazine comprises the remaining total daily dosage amount.

The active ingredient (e.g., eltoprazine) or the composition of the present invention may be used for the manufacture of a medicament for the treatment of at least one of the mentioned disorders, wherein the medicament is adapted to or appropriately prepared for a specific administration as disclosed herein (e.g., to once-a-day, twice-a-day, or three times a day administration). For this purpose the package leaflet and/or the patient information contains corresponding information.

According to the present invention, the dosage form of eltoprazine, or an eltoprazine salt, may be a solid, semisolid, or liquid formulation according to the following.

Eltoprazine may be administered orally, topically, parenterally, or mucosally (e.g., buccally, by inhalation, or rectally) in dosage unit formulations containing conventional non-toxic pharmaceutically acceptable carriers. In another embodiment, eltoprazine may be formulated as a flavored liquid (e.g., peppermint flavor). Eltoprazine may be administered orally in the form of a capsule, a tablet, granules, pellets or the like, or as a semi-solid, or liquid formulation (see Remington's Pharmaceutical Sciences, 20th Edition, by A.R. Gennaro).

For oral administration in the form of a tablet or capsule, eltoprazine may be combined with non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like.

The tablets may be coated with a concentrated sugar solution which may contain e.g., gum arabic, gelatine, talcum, titanium dioxide, and the like. Alternatively, the tablets may be coated with a polymer that dissolves in a readily volatile organic solvent or mixture of organic solvents. In specific embodiments, eltoprazine is formulated in immediate-release (IR) or modified-release (MR) tablets. Immediate release solid dosage forms permit the release of most or all of the active ingredient over a short period of time, such as 60 minutes or less, and make rapid absorption of the drug possible. Modified release solid oral dosage forms permit the sustained release of the active ingredient over an extended period of time in an effort to maintain therapeutically effective plasma levels over similarly extended time intervals and/or to modify other pharmacokinetic properties of the active ingredient. For example, eltoprazine may be formulated in a modified release dosage form (including modified release tablets) to provide a dose of eltoprazine.

For the formulation of soft gelatin capsules, eltoprazine may be admixed with e.g., a vegetable oil or polyethyleneglycol. Hard gelatin capsules may contain granules of the active substances using either the above-mentioned excipients for tablets e.g., lactose, saccharose, sorbitol, mannitol, starches (e.g., potato starch, corn starch or amylopectin), cellulose derivatives or gelatine. Also liquids or semisolids of the drug may be filled into hard gelatine capsules.

Eltoprazine may also be introduced in microspheres or microcapsules, e.g., fabricated from polyglycolic acid/lactic acid (PGLA) (see, e.g., U.S. Patents No. 5,814,344; 5,100,669 and 4,849,222; PCT Publications No. WO 95/11010 and WO 93/07861). Biocompatible polymers may be used in achieving controlled release of a drug, include for example, polylactic acid, polyglycolic acid, copolymers of polylactic and polyglycolic acid, poly(epsilon-caprolactone), polyhydroxybutyric acid, polyorthoesters, polyacetals, polyhydropyrans, polycyanoacrylates, and cross-linked or amphipathic block copolymers of hydrogels.

Formulation of eltoprazine in a semi-solid or liquid form may also be used. Eltoprazine may constitute between 0.1 and 99% by weight of the formulation, more specifically between 0.5 and 20% by weight for formulations intended for injection and between 0.2 and 50% by weight for formulations suitable for oral administration.

In one embodiment of the invention, eltoprazine is administered in a modified release formulation. Modified release dosage forms provide a means for improving patient compliance and for ensuring effective and safe therapy by reducing the incidence of adverse drug reactions. Compared to immediate release dosage forms, modified release dosage forms may be used to prolong pharmacologic action after administration, and to reduce variability in the plasma concentration of a drug throughout the dosage interval, thereby eliminating or reducing sharp peaks.

A modified release dosage form may comprise a core either coated with or containing a drug. The core is then coated with a release-modifying polymer within which the drug is dispersed. The release-modifying polymer disintegrates gradually, releasing the drug over time. Thus, the outer-most layer of the composition effectively slows down and thereby regulates the diffusion of the drug across the coating layer when the composition is exposed to an aqueous environment, i.e. the gastrointestinal tract. The net rate of diffusion of the drug is mainly dependent on the ability of the gastric fluid to penetrate the coating layer or matrix and on the solubility of the drug itself.

In another embodiment of the invention, eltoprazine is formulated in an oral, liquid formulation. Liquid preparations for oral administration may take the form of, for example, solutions, syrups, emulsions or suspensions, or they may be presented as a dry product for reconstitution with water or other suitable vehicle before use. Preparations for oral administration may be suitably formulated to give controlled or postponed release of the active compound.

For oral administration in liquid form, eltoprazine may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms. For example, solutions may contain from about 0.2% to about 20% by weight of eltoprazine, with the balance being sugar and mixture of ethanol, water, glycerol and propylene glycol. Optionally, such liquid formulations may contain coloring agents, flavoring agents, saccharine and carboxymethyl-cellulose as a thickening agent or other excipients.

In another embodiment, a therapeutically effective amount of eltoprazine is administered in an oral solution containing a preservative, a sweetener, a solubilizer, and a solvent. The oral solution may include one or more buffers, flavorings, or additional excipients. In a further embodiment, a peppermint or other flavoring is added to the eltoprazine oral liquid formulation.

For administration by inhalation, eltoprazine may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

Solutions for parenteral applications by injection may be prepared in an aqueous solution of the compound or of a water-soluble pharmaceutically acceptable salt of the active substances, for example in a concentration of from about 0.5% to about 10% by weight. These solutions may also contain stabilizing agents and/or buffering agents and may conveniently be provided in various dosage unit ampoules.

The formulations of the invention may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection may be presented in unit dosage form, e.g., in ampoules or in multi-dose containers, with an added preservative. Alternatively, the active ingredient may be in powder form for reconstitution with a suitable vehicle, e.g., sterile pyrogen-free water, before use.

The invention also provides a pharmaceutical pack or kit comprising one or more containers containing eltoprazine and, optionally, more of the ingredients of the formulation. In a specific embodiment, eltoprazine is provided as an oral solution for administration with the use of a 2-teaspoon capacity syringe (dosage KORC®). Each oral syringe has hatch marks for measurement, with lines on the right side of the syringe (tip down) representing tsp units, and those on the left representing ml units.

The optimal therapeutically effective amount may be determined experimentally, taking into consideration the exact mode of administration, form in which the drug is administered, the indication toward which the administration is directed, the subject involved (e.g., body weight, health, age, sex, etc.), and the preference and experience of the physician or veterinarian in charge.

In certain embodiments, the invention relates to the use of eltoprazine in the preventive treatment of a patient receiving L-DOPA treatment, i.e. where the L-DOPA-treated patient is still free of symptoms.

In one such embodiment, eltoprazine is for administration to a patient, who receives L-DOPA as first-line treatment of PD, particularly within about 9, about 6, about 4, about 3 years or about 2 years from start of L-DOPA treatment. In a particular embodiment, the patient is at the age of at least 60, 65 or 70 years.

In another such embodiment, eltoprazine is for administration to a patient, who either receives L-DOPA in combination with one or more dopamine receptor agonists, or as second-line treatment of PD after prior treatment with one or more dopamine receptor agonists, particularly within about 9, about 6, about 4, about 3 years or about 2 years from start of L-DOPA treatment. Alternatively, the patient is treated with eltoprazine from the point in time on, where such L-DOPA combination or second-line treatment is started.

Sometimes a patient is more interested in reducing the periods of insufficient motility ("off-phase") than in the reduction of dyskinesia ("on-phase"). The dosage of a treatment with L-DOPA and/or dopamine agonists, however, can only be increased as long as the occurrence of LID is still bearable. Therefore, in another embodiment, eltoprazine is used to keep the LID symptoms at a stable level (rather than reducing them), thereby enabling to increase the dosage of a treatment with L-DOPA and/or dopamine agonists and as such decreasing the off-phase. Thus, this embodiment relates to a method of using eltoprazine or a pharmaceutically acceptable salt thereof for, or the use of eltoprazine or a pharmaceutically acceptable salt thereof for the manufacture of a medicament for, maintaining the level of L-DOPA-induced dyskinesia in a patient that is treated with an increased amount of L-DOPA and/or dopamine receptor agonists. In the context of the present invention, the term "maintaining the level" has the meaning that dyskinetic events are kept at a level that shows about the same grade and/or frequency of dyskinetic events as at beginning of eltoprazine treatment, and the term "increased amount of L-DOPA and/or dopamine receptor agonists" has the meaning that the patient is treated with a higher amount of L-DOPA when compared with the amount at the beginning of eltoprazine treatment. Thus, the invention relates to a method/composition/use, wherein
a) an effective amount of eltoprazine or a pharmaceutically acceptable salt thereof is administered to a subject;
b) such effective amount is selected to that effect that the L-DOPA induced dyskinesia is kept at a level which was present in the subject without the administration of such effective amount; and
c) the dosage of L-DOPA is increased.

Dosage units for rectal application may be solutions or suspensions or may be prepared in the form of suppositories or retention enemas comprising eltoprazine in a mixture with a neutral fatty base, or gelatin rectal capsules comprising the active substances in admixture with vegetable oil or paraffin oil.

Toxicity and therapeutic efficacy of the compositions of the invention may be determined by standard pharmaceutical procedures in experimental animals, e.g., by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

Suitable daily doses of the active ingredient of the invention (eltoprazine) in therapeutic treatment of humans are within the range from about 5 mg to about 150 mg per day, such as from about 5 mg to about 120 mg, from about 5 mg to about 100 mg, or from about 5 mg to about 75 mg, or from about 5 mg to about 60 mg, or from about 5 mg to about 50 mg, or from about 10 mg to about 20 mg, or from about 10 mg to about 15 mg, such as 10mg or 15 mg or 20 mg or 30 mg or 40 mg or 60mg or 80mg, per day. For example the daily dose may be body weight-adjusted such as 40 mg/day up to 80 kg body weight or 60 mg/day for patients with a body weight of ≥ 80 kg. In modified release formulations the amounts of active ingredient per day could also be higher due to reduced bioavailablitity, e.g. up to 200 mg/day. An equimolar amount of another pharmaceutically acceptable salt, a solvate, a conjugate or a derivative thereof, such as eltoprazine hydrochloride, is also suitable. For pediatric subjects aged 4-14, eltoprazine may be administered as an oral, liquid dosage form, at reduced amounts, for example from about 1 mg/day, up to about 5 mg/day.

In particular embodiments, the daily dosage of eltoprazine is between about 10 and 20 mg/day.

The daily doses indicated herein may be administered, for example, as one or two dosing units once, twice or three times per day. Suitable doses per dosage unit may therefore be the daily dose divided (for example, equally) between the number of dosage units administered per day, and will thus typically be about equal to the daily dose or one half, one third, one quarter or one sixth thereof. Dosages per dosage unit may thus be calculated from each daily dosage indicated herein. A daily dose of 5 mg, for example may be seen as providing a dose per dosage unit of, for example, about 5 mg, 2.5 mg, 1.67 mg, 1.25 mg and 0.83 mg, depending upon the dosing regimen chosen. Correspondingly, a dosage of 50 mg per day corresponds to dosages per dosing unit of, for example, about 50 mg, 25 mg, 16.7 mg, 12.5 mg, and 8.33 mg for corresponding dosing regimens. In other embodiments, especially in cases where only two daily doses are administered, an unequal split of the first and the second dosage is envisaged. In such cases, for example, the first dosage comprises about 55 to 65% of the total daily dosage. For example, a daily dosage of 10 mg is split into a first dosage of 6 mg and a second dosage of 4 mg. In cases, in which more than two administration per day are envisaged, the dosage might be split also unequally, wherein the second and further dosages are reduced in comparison to the dosage before. For example, if three dosages per day are administered, the first dosage could be about one half of the total daily dosage, i.e. between about 40% to 60%, the second dosage could be about one third of the total daily dosage, i.e. between about 20% to 40%, and the third dosage could be about one sixth of the total daily dosage, i.e. between about 5% to 20%. For example, a daily dosage of 10 mg could be split into a first dosage of 6 mg, a second dosage of 3 mg and a third dosage of 1 mg.

In certain such embodiments, the treatment is the treatment of diphasic dyskinesia (DID).

Treatment duration may be short-term, e.g., several weeks (for example 8-14 weeks), or long-term until the attending physician deems further administration no longer is necessary.

Eltoprazine may be administered as a single antidyskinetic agent in combination with dopamine replacement therapy (e.g. L-DOPA) and/or therapy with dopamine receptor agonists (e.g. apomorphine) for the treatment of L-DOPA-induced dyskinesias. In particular embodiments, L-DOPA is administered together with a decarboxylase inhibitor, including but not limited to benserazide (e.g. in Levodopa comp. B STADA^{®}, Levopar^{®} Madopar^{®}, PK-Levo^{®}, or Restex^{®}), or carbidopa (e.g. in Nacom^{®}, Dopadura C^{®}, Levobeta C^{®}, or Stalevo^{®}).

The term "combination" applied to active ingredients is used herein to define two separate pharmaceutical compositions, each comprising an active agent (e.g. eltoprazine, and another pharmaceutical composition comprising another agent prescribed for the treatment of motor disorders such as Parkinson disease), to be administered conjointly.

Within the meaning of the present invention, the term "conjoint administration" is used to refer to administration of eltoprazine, and a second active agent simultaneously in different compositions, or sequentially. For the sequential administration to be considered "conjoint", however, eltoprazine, and the second active agent must be administered separated by a time interval, which still permits the resultant beneficial effect for treating L-DOPA-induced dyskinesias in a mammal.

### EXAMPLES

The following examples illustrate the invention without limiting its scope.

### EXAMPLE 1: Effect of eltoprazine on L-DOPA Induced Dyskinesia in the 6-hyrdroxydopamine (6-OHDA)-lesioned rat model of Parkinson's disease

### Materials and Methods

### Animals

Male Sprague Dawley rats (Elevage Janvier, Le Genest Saint Isle, France) weighing between 220 and 250 g at the beginning of the study are used in these experiments. They are housed under a 12-hr light/dark cycle with free access to standard pelleted food and tap water. Animal treatment and experimental procedures are approved by local ethical committees (Regierungspräsidium Darmstadt; Germany).

### Dopamine-denervating lesions

Dopamine-denervating lesions are performed on rats anaesthetized with a 5:1 mixture of ketamine and xylazine (1 ml/kg, i.p.). All rats receive unilateral injection of 6-hydroxydopamine (6-OHDA-HCl) (3 µg/µL in 0.02% ascorbate-saline) into the right ascending DA fibre bundle at the following coordinates (in mm relative to bregma and the dural surface): (1) A = - 4.4, L = -1.2, V = -7.8, tooth bar -2.3 (7.5 µg deposit); (2) A = -4.0, L = -0.75, V = -8.0, tooth bar = +3.4 (6 µg deposit). Injections are performed at the rate of 1 µL/min (allowing an additional 3 min before retracting the needle) using a 10-µL Hamilton microsyringe with a 26-gauge steel cannula. In order to assess the efficacy of the lesions, all rats are tested for amphetamine-induced rotation 2 weeks after the 6-OHDA injections. The animals' turning behaviour is recorded in an automated rotometer (TSE Rotameter System, TSE -Systems GmbH, Bad Homburg, Germany) over a 90-min period after the intraperitoneal (i.p.) injection of 2.5 mg/kg dexamphetamine sulphate dissolved in saline. Only the rats showing rotational scores > 5 net full turns/min in the direction ipsilateral to the lesion are selected for the study.

### Experimental Design and Drug Treatment

At 6-8 weeks post-lesion, rats are treated for 21 days with a single daily i.p. injection of 6 mg/kg of L-DOPA mixed with 15 mg/kg of the peripheral DOPA-decarboxylase inhibitor benserazide hydrochloride or with saline (vehicle controls). L-DOPA and benserazide are dissolved in a physiological saline solution. Chronic treatment with this dose of L-DOPA has been shown to induce gradual development of dyskinetic-like movements in 6-OHDA-lesioned rats. After ca. 3 weeks of the daily treatment, rats are injected 30 minutes before the evaluation of abnormal involuntary movement (AIM)s with different doses of eltoprazine hydrochloride (0.08, 0.3, 1.25 and 5 mg/kg, s.c.), followed by L-DOPA (L-DOPA 6 mg/kg, and benserazide 15 mg/kg), i.p., 10 minutes before the beginning of the test.

### Behavioral Test

In order to evaluate the severity of LID, AlMs are recorded every second day as described by Cenci et al. (1998). Cenci MA, Lee CS, Björklund A. L-DOPA-induced dyskinesia in the rat is associated with striatal overexpression of prodynorphin- and glutamic acid decarboxylase mRNA. Eur J Neurosci 1998 10:2694-2706. Briefly, rats are observed individually for 1 min every 10th minute during 3 h following a daily L-DOPA dose. Repetitive movements affecting the side of the body contralateral to the lesion that could not be ascribed to any normal behavioural pattern are classified into four different subtypes: locomotive AlMs, i.e., increased locomotion with contralateral side bias; axial dystonia, i.e., contralateral twisted posturing of the neck and upper body; orolingual AlMs, i.e., stereotyped jaw movements and contralateral tongue protrusion; and forelimb dyskinesia, i.e., repetitive jerks of the contralateral forelimb, sometimes combined with grabbing movements of the paw. Each rat is scored on a severity scale from 0 to 4 based on its frequency and persistence (1 = occasional; 2 = frequent; 3 = continuous but interrupted by sensory distraction; 4 = continuous, severe and not interrupted by sensory distraction). The axial, orolingual and forelimb (AOL) AlMs are presented together as a mean (mean AIM score) per time point. In this experiment, L-DOPA (6.25mg/kg + Benserazide 15mg/kg) is injected alone or in combination with eltoprazine at 0.08, 0.3, 1.25 and 5 mg/kg.

In order to evaluate the effect of eltoprazine on parkinsonian symptoms and its interaction with L-DOPA, rotational behaviour is evaluated. The numbers of ipsilateral and contralateral turns are recorded in an automated rotometer (TSE Rotameter System, TSE -Systems GmbH, Bad Homburg, Germany) over a period of 180 min. For the effect on parkinsonian symptoms, eltoprazine is administered at 0.3, 1.25 and 5 mg/kg 30 minutes before the test.

### Statistical Analysis

ANOVA and two-way ANOVA are used to evaluate the significance of the results. Post hoc Tukey test is performed where appropriate.

### Neurochemical profile of eltoprazine

### 5-HT1a and 5-HT1b receptors

Eltoprazine was tested for activity on the human serotonin 5-HT1 a and 5-HT1 b receptors using a GTPγS assay. Recombinant membranes obtained from CHO-K1 cells expressing either the 5-HT1a or 5-HT1 b receptors were mixed with GDP (volume:volume) and incubated for at least 15 min on ice. In parallel, GTPγ[35S] was mixed with the beads (volume:volume) just before starting the reaction. The following reagents were successively added in the wells of an Optiplate (Perkin Elmer): 50 µl of test compound, 20µl of the membranes:GDP mix, 10 µl of assay buffer (for agonist testing) and 20 µl of the GTPγ[35S]:beads mix. The plates were covered with a top seal, shaken on an orbital shaker for 2 min, and then incubated for 1 hour at room temperature. Then the plates were centrifuged for 10 min at 2000 rpm, incubated at room temperature 1 hour and counted for 1min/well with a PerkinElmer TopCount reader.

### 5-HT2a, 5-HT2b and 5-HT2c non-edited receptor

Aequorin cell lines expressing the 5-HT2a, 5-HT2b and 5-HT2c non-edited (ne) recombinant receptors were used to evaluate the functional activity of eltoprazine. Aequorin cells grown 18 hours prior to the test in media without antibiotics were detached by gentle flushing with PBS-EDTA (5 mM EDTA), recovered by centrifugation and resuspended in "assay buffer" (DMEM/HAM's F12 with HEPES + 0.1% BSA protease free). Cells were incubated at room temperature for at least 4h with Coelenterazine h (Molecular Probes). The reference agonist used were 5-HT and α-methyl-5-HT. For agonist testing, 50µl of cell suspension were injected on 50µl of test compound or reference agonist plated in a 96-well plate. The resulting emission of light was recorded using the Hamamatsu Functional Drug Screening System 6000 (FDSS 6000).

### Results

**Figure 1** illustrates the potency of eltoprazine on different serotoninergic receptor activated at relevant plasma concentration.

**Figure 2** illustrates the effect of increasing doses of eltoprazine on AIM scores in 6-OHDA-lesioned rats. The data are expressed as % of AIM scores compared to L-DOPA - vehicle-treated animals. * indicates a significant difference with p<0.05 between L-DOPA - vehicle-treated animals (ANOVA).

This result demonstrates that eltoprazine significantly reverses, in a dose-dependent manner, the AlMs that arise in 6-OHDA-lesioned rats after 21 days of treatment with L-DOPA. The effect of amantadine (40mg/kg) and buspirone (1mg/kg) in the dyskinetic rat model are presented on the same graph for comparison.

**Figure 3** illustrates the effect of eltoprazine on the turning behaviour induced by high dose of L-DOPA in 6-OHDA-lesioned rats. * indicates a significant difference with p<0.05 versus vehicle-treated animals, ^{#} indicates a significant different with p<0.05 versus eltoprazine-treated animals (2-way ANOVA followed by Tukey's Post hoc test).

This result demonstrates that eltoprazine does not interfere with the antiparkinsonian effect of L-DOPA.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

All patents, applications, publications, test methods, literature, and other materials cited herein are hereby incorporated by reference.

## Claims

1. A composition comprising eltoprazine or a pharmaceutically acceptable salt thereof for the treatment or prevention of L-DOPA-induced dyskinesia.

2. Use of eltoprazine or a pharmaceutically acceptable salt thereof for the manufacture of a medicament for the treatment or prevention of L-DOPA-induced dyskinesia.

3. The composition according to Claim 1, or the use according to Claim 2, wherein the substance eltoprazine is eltoprazine hydrochloride.

4. The composition/use according to any of the preceding claims, wherein eltoprazine is for administration in a range from about 5 mg to about 75 mg/day, or in a range from about 5 mg to about 60 mg/day, or in a range from about 5 mg to about 50 mg/day, or in a range from about 5 mg to about 40 mg/day, or in a range from about 5 mg to about 20 mg/day, or in a range from about 5 mg to about 15 mg/day, particularly, wherein eltoprazine is for administration in a range from about 10 mg to about 15 mg/day.

5. The composition/use according to Claim 4, wherein eltoprazine is for administration at about 5 mg/day, or eltoprazine is for administration at about 10 mg/day, or eltoprazine is for administration at about 15 mg/day, or eltoprazine is for administration at about 20 mg/day, or eltoprazine is for administration at about 40 mg/day, or eltoprazine is for administration at about 60 mg/day.

6. The composition/use according to any of the preceding claims, wherein eltoprazine or a pharmaceutically acceptable salt thereof is for administration once a day, twice a day (b.i.d.), or three times a day, particularly, wherein eltoprazine or a pharmaceutically acceptable salt thereof is for administration twice a day (b.i.d.), particularly, wherein the daily dosage for said administration twice a day (b.i.d.) is split into a first dose of about 55 to 65% of the total daily dosage amount, and a second dose comprising the remaining total daily dosage amount, particularly, wherein the second dosage is administered at about lunchtime.

7. The composition/use according to any of the preceding claims, wherein the subject to be treated suffers from a movement disorder, particularly, wherein the movement disorder is parkinsonism, particularly, wherein the parkinsonism is idiopathic (PD).

8. The composition/use according to Claim 7, wherein the parkinsonism is idiopathic (PD), and wherein the subject is undergoing long-term treatment with L-DOPA therapy indicated for the treatment of PD.

9. The composition/use according to Claim 7 or 8, wherein the parkinsonism is idiopathic (PD), and wherein the subject is undergoing treatment with dopamine receptor agonists.

10. The composition/use according to any of claims 1 to 9, wherein said treatment or prevention comprises the additional administration with at least one additional pharmaceutical agent.

11. The composition/use according to Claim 10, wherein the additional pharmaceutical agent(s) is/are selected from the group of (i) an agent which has been shown to be effective for the treatment of PD, and (ii) a decarboxylase inhibitor such as benserazide and carbidopa.

12. The composition/use according to any of the preceding claims in the form of an oral formulation.

13. The composition/use according to any of the preceding claims, wherein said effective amount of eltoprazine or a pharmaceutically acceptable salt thereof is administered at least about 10 minutes before the administration of a therapeutically effective amount of L-DOPA.

14. The composition/use according to any of the preceding claims, wherein said method/composition/use is for the prevention of L-DOPA-induced dyskinesia, wherein eltoprazine or a pharmaceutically acceptable salt thereof is administered to a subject receiving L-DOPA, wherein said subject is still free of symptoms of L-DOPA induced dyskinesia, and the treatment with said composition/use is started at the latest 9 years after the beginning of said L-DOPA treatment, particularly, wherein (i) said subject receives L-DOPA as first-line treatment of Parkinson's disease; or (ii) wherein said subject receives L-DOPA either in combination with a dopamine agonist or as a second-line treatment of Parkinson's disease.

15. The composition/use according to any of Claims 1 to 14, wherein
a) an effective amount of eltoprazine or a pharmaceutically acceptable salt thereof is administered to said subject;
b) said effective amount is selected such that the L-DOPA induced dyskinesia is kept at a level which was present in said subject without the administration of said effective amount; and
c) the dosage of L-DOPA is increased.
